(19) Europäisches Patentamt
European Patent Office
Office européen des brevets

(11) Numéro de publication : **0 007 285**
**B1**

(12) **FASCICULE DE BREVET EUROPÉEN**

(45) Date de publication du fascicule du brevet : **03.02.82**

(21) Numéro de dépôt : **79420031.1**

(22) Date de dépôt : **04.07.79**

(51) Int. Cl.[3] : **C 07 C 39/11**, C 07 C 37/01, C 07 F 5/04

(54) **Procédé de préparation des alcools orthohydroxybenzyliques.**

(30) Priorité : **11.07.78 FR 7821591**

(43) Date de publication de la demande : **23.01.80 (Bulletin 80/02)**

(45) Mention de la délivrance du brevet : **03.02.82 Bulletin 82/05**

(84) Etats contractants désignés : **BE CH DE GB IT NL**

(56) Documents cités : **FR - A - 1 328 945**

(73) Titulaire : **RHONE-POULENC INDUSTRIES Brevets Pharma 25 Quai Paul Doumer F-92408 Courbevoie Cedex (FR)**

(72) Inventeur : **Le Ludec, Joel 11, rue de Boyer F-69005 Lyon (FR)**

(74) Mandataire : **Rioufrays, Roger et al RHONE-POULENC INDUSTRIES Centre de Recherches des Carrières Service Brevets F-69190 Saint-Fons (FR)**

**EP 0 007 285 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation des alcools orthohydroxybenzyliques

La présente invention a pour objet un procédé de préparation d'alcools orthohydroxybenzyliques, et plus particulièrement de l'alcool orthohydroxybenzylique, communément nommé saligénol, par condensation du formaldéhyde ou d'un composé générateur de formaldéhyde avec des esters dérivés de l'acide borique et du phénol ou des phénols substitués.

Les alcools orthohydroxybenzyliques sont des produits intermédiaires recherchés pour la préparation des acides orthohydroxyméthylphénylacétiques utilisés comme régulateur de croissance des plantes. Le saligénol lui-même est un produit industriel important tant pour les propriétés pharmacologiques que comme intermédiaiire pour la synthèse d'insecticides.

Le procédé industriel le plus intéressant pour la fabrication des alcools orthohydroxybenzyliques consiste à faire réagir le formaldéhyde ou un de ses dérivés avec un métaborate d'aryle : cf. brevet français 1 328 945. Ce procédé conduit au saligénol avec des rendements voisins de 65 % exprimés par rapport au phénol et au formaldéhyde mis en œuvre dans la réaction. En dépit de son intérêt, on constate que ce procédé n'est pas dénué d'inconvénient au plan économique. En effet dans un tel procédé, le formaldéhyde non transformé en saligénol est perdu sous forme de sous-produits et/ou ne peut être récupéré à partir de la masse réactionnelle. De son côté l'acide borique bien que non transformé dans le processus réactionnel est également perdu car sa récupération exigerait la mise en œuvre de techniques d'isolement coûteuses. La perte de ces deux produits contribue par conséquent à grever le prix de revient du saligénol. La présente invention se propose de pallier les inconvénients du procédé décrit dans le brevet français 1 338 945 et plus particulièrement d'améliorer notablement le rendement en alcools orthohydroxybenzyliques par rapport au phénol et au formaldéhyde et leur productivité par rapport à l'acide borique mis en œuvre.

Plus spécifiquement la présente invention concerne un procédé de préparation d'alcools orthohydroxybenzyliques par réaction d'esters d'acide borique et de phénols avec du formaldéhyde ou un corps générateur de formaldéhyde conduisant à la formation d'esters boriques d'alcools orthohydroxybenzyliques puis décomposition de ces derniers pour libérer les alcools orthohydroxybenzyliques, caractérisé en ce que les esters boriques de phénols utilisés pour la condensation sont obtenus par réaction d'au moins 1,1 mole d'un phénol avec une mole d'acide borique.

On a constaté de façon surprenante que l'utilisation pour la condensation avec le formaldéhyde d'esters boriques de phénols obtenus par réaction d'au moins 1,1 mole d'un phénol avec 1 mole d'acide borique permet d'améliorer sensiblement le rendement en alcools orthohydroxybenzyliques par rapport au phénol transformé et de façon notable le rendement en alcools hydroxybenzyliques par rapport au formaldéhyde engagé dans la réaction. Il en résulte parallèlement une diminution de la consommation d'acide borique par kilogramme d'alcools benzyliques fabriqués.

Les esters boriques de phénols obtenus par réaction d'au moins 1,1 mole de phénols avec 1 mole d'acide borique — que l'on dénommera par la suite, pour raison de commodité, « borate d'aryle » — sont des mélanges complexes formés :

— de métaborates de phénols de formule :

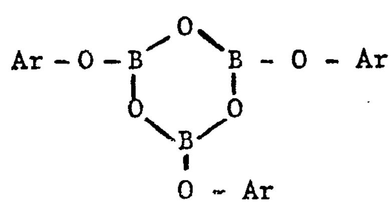

(I)

— de pyroborates de phénols :

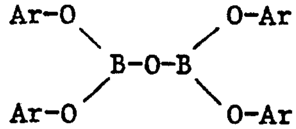

(II)

— d'orthoborates de phénols :

$$(Ar{-}O{-})_3B \qquad (III)$$

— de borates acides de phénols :

$$(Ar{-}O{-})_2{-}B{-}OH \qquad (IV)$$

(dans lesquelles Ar représente un radical phényle substitué ou non, tel qu'il sera défini plus loin), contenant éventuellement le phénol en excès. La proportion de chacun de ces dérivés de l'acide borique dans le mélange d'estérification varie évidemment en fonction du rapport molaire phénol/acide borique et/ou du taux d'estérification. Ainsi, pour des rapports molaires phénol/acide borique compris entre 1,1 et 1,5 le mélange est principalement formé de métaborates et pour des rapports égaux ou voisins de 3 les orthoborates sont pratiquement les seuls composants du mélange. Lors de la préparation des borates d'aryle, on a constaté qu'il n'est pas nécessaire de transformer en borate la totalité du phénol mis en œuvre, notamment lorsque le rapport molaire phénol/acide borique est voisin de 3 pour obtenir de bons rendements en alcools orthohydroxybenzyliques ; il est donc possible de limiter le taux d'estérification du phénol considéré sans cependant que ce taux soit inférieur à 70 % et de préférence 80 % du phénol mis en œuvre. On obtient dans ce cas un mélange de borates d'aryle contenant le phénol non transformé, des borates acides de formule (IV) et de l'orthoborate d'aryle.

La préparation des borates d'aryle est réalisée, conformément aux procédés connus, par réaction d'un phénol avec l'acide borique dans un solvant formant un azéotrope avec l'eau de la réaction d'estérification. Cette dernière est éliminée au fur et à mesure de sa formation par distillation azéotropique. Comme solvant convenant à la préparation des borates d'aryle on peut citer des hydrocarbures aromatiques tels que le benzène, le toluène et le xylène. Tout autre solvant inerte permettant la distillation azéotropique de l'eau peut être utilisé.

La condensation du formaldéhyde avec le borate d'aryle peut être conduite directement sur la solution de borate d'aryle anhydre ainsi obtenue, éventuellement après dilution par une quantité additionnelle du solvant choisi. La quantité de formaldéhyde mise en œuvre est de préférence de 1 mole par mole d'acide borique bien que l'on puisse s'écarter quelque peu de cette proportion (elle peut être comprise par exemple entre 0,9 et 1,1 mole par mole d'acide borique), mais sans que cela se traduise par un avantage particulier. Lorsqu'on utilise un générateur de formaldéhyde (polymères du formaldéhyde par exemple) la quantité est évidemment calculée pour que la quantité de formaldéhyde disponible à la réaction soit de 1 mole par mole d'acide borique.

On pourrait, sans sortir du cadre de la présente invention, conduire la condensation borate de phényle/formaldéhyde dans un solvant différent de celui utilisé à la phase de préparation du borate d'aryle mais cela complique le procédé sans apporter d'avantage.

La température de la condensation du formaldéhyde ou de son générateur avec le phénol peut être comprise entre 20 et 120 °C et, de préférence entre 40 et 100 °C.

La masse réactionnelle de condensation est un mélange complexe de borates d'alcools orthohydroxybenzyliques et de borates mixtes d'aryle et d'alcools orthohydroxybenzyliques de composition variant avec celles du mélange de borates d'aryle utilisé comme produit de départ. Quelle que soit la composition de cette masse réactionnelle la libération des alcools orthohydroxybenzyliques à partir des produits de condensation peut être réalisée conformément aux procédés décrits dans le brevet français 1 328 945, c'est-à-dire par saponification, alcoolyse ou hydrolyse. Le procédé de saponification convient tout particulièrement bien car il permet notamment dans les cas où le rapport phénol/acide borique devient important de récupérer aisément les phénols en excès qui peuvent être recyclés à une nouvelle opération de préparation de borates d'aryle. Cette technique convient tout spécialement bien lorsque le rapport phénol/acide borique est compris entre 1,5 et 3, ce qui implique la récupération des phénols. Pour mener à bien une telle récupération du phénol et sa séparation des alcools orthohydroxybenzyliques, il importe dans une première étape de conduire la saponification avec une quantité d'agent alcalin en solution aqueuse (notamment hydroxydes de sodium ou de potassium en solution aqueuse) calculée de façon à provoquer la formation à partir des borates d'alcools orthohydroxybenzyliques des complexes de formule :

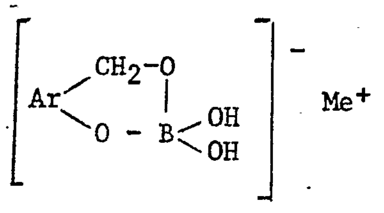

(V)

dans laquelle Me représente un métal alcalin, lesquels complexes sont solubles dans l'eau, sans entraîner la formation des phénates alcalins à partir des phénols libérés lors de la saponification. Dans ces conditions les phénols libérés restent en solution dans le solvant organique utilisé lors de la phase d'estérification et de condensation et le sel complexe de formule (V) dérivé de l'alcool orthohydroxybenzylique formé, passe dans la phase aqueuse. Les phases organique et aqueuse sont alors séparées par décantation. La phase organique contenant le phénol en excès peut être directement réutilisée pour une nouvelle opération. Dans une deuxième étape la phase aqueuse peut de son côté être traitée de diverses manières pour libérer l'alcool orthohydroxybenzylique à partir du complexe de formule (V). On peut par exemple traiter la solution aqueuse par un acide minéral ou encore déplacer l'alcool orthohydroxybenzylique par action d'un composé ayant un pouvoir complexant supérieur à celui de cet alcool, par exemple un polyol tel que le mannitol et le sorbitol qui forment avec l'acide borique des complexes très solubles dans l'eau ; les alcools orthohydroxybenzyliques libérés sont extraits par un solvant approprié. On peut également traiter la phase aqueuse après séparation de la phase organique contenant le phénol, par une solution aqueuse alcaline de façon à libérer l'alcool orthohydroxybenzylique sous forme de sel alcalin. On récupère alors une solution aqueuse de borate alcalin et de sel alcalin de l'alcool orthohydroxybenzylique à partir de laquelle l'alcool peut être récupéré par extraction après acidification ou qui peut être utilisée directement pour la préparation de dérivés des alcools orthohydroxybenzyliques. On peut par exemple procéder à l'oxydation de ces derniers sous forme de sels alcalins par l'oxygène ou l'air en les hydroxybenzaldéhydes correspondants.

La quantité de base alcaline à mettre en jeu lors de la première étape de saponification afin de séparer le phénol en excès à la fin de la condensation borate d'aryle/formaldéhyde est d'au plus 1,2 mole par mole d'acide borique et de préférence 1 mole par mole et d'au moins 0,8 et de préférence 0,9 mole de base alcaline par mole d'acide borique. La quantité convenable peut être aisément déterminée dans chaque cas particulier au moyen d'essais simples.

La quantité de base alcaline mise en œuvre lors de la deuxième étape de saponification est généralement comprise entre 0,8 et 1,5 mole par mole d'acide borique initialement mis en œuvre.

Les phénols qui conviennent à la mise en œuvre de la présente invention peuvent être représentés par la formule générale :

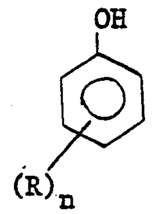

(VI)

dans laquelle n est 0 ou un nombre entier de 1 à 3 et R représente : un radical alcoyle ayant de 1 à 12 atomes de carbone et de préférence de 1 à 4 tels que méthyle, éthyle, propyles, butyles, hexyles, octyles ; un radical alcoyloxyle ayant de 1 à 12 et de préférence de 1 à 4 atomes de carbone tels que les groupes méthoxy, éthoxy, propoxy, butoxy ; un atome d'halogène tel que le chlore et le brome. Lorsque n est supérieur à 1 l'une au moins des positions en ortho du groupe phénolique doit être libre. Les phénols de formule (VI) donnent naissance à des alcools orthohydroxybenzyliques de formule générale

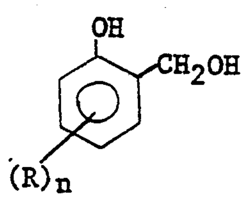

(VII)

dans laquelle R et n ont la signification donnée précédemment.

Comme exemples de phénols de formule (VI) on peut citer le phénol, les crésols, le xylénol-2,3, le xylénol-3,4, les monoéthylphénols, le méthoxy-2 phénol, le méthoxy-3 phénol, le méthoxy-4 phénol, le diméthoxy-2,3 phénol, l'éthoxy-2 phénol, l'éthoxy-4 phénol, les monochlorophénols.

Le procédé selon l'invention convient tout particulièrement bien à la préparation du saligénol à partir du phénol.

Les exemples qui suivent illustrent l'invention et montrent comment elle peut être mise en pratique.

Exemple 1

Dans un ballon de verre de 1 000 ml muni d'une agitation centrale, d'un thermomètre, d'un dispositif de chauffage et d'un système de séparation de l'eau entraînée par azéotropie on charge :

— 155,92 g de phénol à 98,6 % (1,634 mole)
— 33,48 g d'acide borique à 100 % (0,542 mole)
— 40 ml de toluène

et chauffe à l'ébullition en agitant jusqu'à ce que la quantité d'eau éliminée par distillation azéotropique corresponde à 82 % de la quantité d'eau résultant de l'estérification complète. La température dans la masse réactionnelle atteint 160 °C.

Après refroidissement vers 100°, la solution toluénique contenant principalement de l'orthoborate de phényle ainsi obtenue est diluée avec 200 ml de toluène anhydre puis portée à 90 °C.

On charge alors du paraformaldéhyde (16,94 g à 95,9 % soit 0,542 mole) en 30 mn et poursuit

l'agitation à la même température pendant 1 h 30 mn.

La masse réactionnelle est alors refroidie vers 20° puis coulée en 20 mn dans de l'eau glacée (290 g) sous bonne agitation. A ce mélange on ajoute 123,16 g de soude diluée à 17,59 % en poids (soit 0,542 mole de NaOH) et agite 20 mn à température ordinaire. Après arrêt de l'agitation, on laisse décanter.

La phase toluénique supérieure est récupérée.

La phase aqueuse est lavée avec du toluène (2 fois 100 ml).

Les couches toluéniques sont rassemblées : elles contiennent le phénol en excès que l'on dose par chromatographie en phase liquide, soit 1,087 mole. Le taux de transformation du phénol s'élève donc à 33,5 %.

A la phase aqueuse débarrassée du phénol on ajoute 165,05 g d'une solution aqueuse de soude à 17,59 % en ppids (soit 0,72 mole de NaOH) : cette solution contient le borate de sodium et le saligénate de sodium que l'on dose par chromatographie en phase liquide dans une colonne de diamètre intérieur de 4 mm et de 15 cm de longueur garnie d'une phase d'octadécyle triméthoxy silane greffé sur silice de 5 mm de granulométrie (produit vendu par la Société MERCK, sous la référence RP 18). L'éluant est constitué par une solution hydroalcoolique obtenue par mélange de 25 % en volume d'éthanol et de 75 % en volume d'une solution aqueuse tamponnée à pH = 3,4 obtenue par dilution à 1 000 ml d'un mélange de 0,923 g d'acétate de sodium trihydraté et de 7 ml d'acide acétique. On dose de cette façon 0,528 mole de saligénol ce qui correspond à un rendement par rapport au formaldéhyde mis en œuvre de 97,6 %. Le rendement par rapport au phénol non récupéré s'élève à 97 %.

Exemples 2 à 4

On a répété l'exemple 1 en faisant varier le rapport molaire phénol/acide borique. On a obtenu les résultats suivants :

| Exemples | Phénol/ $H_3BO_3$ | Taux de transformation phénol % | Rendement en saligénol/ au formaldéhyde engagé % | Rendement en saligénol/ phénol non récupéré % |
|---|---|---|---|---|
| 2 | 1,2 | 79 | 82,5 | 87 |
| 3 | 1,5 | 67 | 91,4 | 91 |
| 4 | 2 | 51,5 | 93,3 | 90,6 |

Note : Le taux d'estérification a été de 97, 93 et 98 % respectivement aux exemples 2, 3 et 4.

**Revendications**

1. Procédé de préparation d'alcools orthohydroxybenzyliques par réaction d'esters d'acide borique et de phénols avec du formaldéhyde ou un corps générateur de formaldéhyde, avec formation d'esters boriques d'alcools orthohydroxybenzyliques, puis décomposition de ces derniers pour libérer les alcools orthohydroxybenzyliques, caractérisé en ce que les esters boriques de phénols utilisés pour la condensation sont obtenus par réaction d'au moins 1,1 mole d'un phénol avec une mole d'acide borique.

2. Procédé de préparation d'alcools orthohydroxybenzyliques selon la revendication 1, caractérisé en ce que les borates de phénols ont été obtenus par réaction de 1,1 à 3 moles d'un phénol avec 1 mole d'acide borique.

3. Procédé de préparation d'alcools orthohydroxybenzyliques selon l'une quelconque des revendications 1 à 2, caractérisé en ce que les borates de phénols sont en solution dans le solvant utilisé lors de l'estérification du phénol par l'acide borique.

4. Procédé de préparation d'alcools orthohydroxybenzyliques selon la revendication 3, caractérisé en ce que le solvant est un hydrocarbure aromatique.

5. Procédé de préparation d'alcools orthohydroxybenzyliques selon l'une quelconque des revendications 1 à 4, caractérisé en ce que la quantité de formaldéhyde engagée est voisine de 1 mole par mole d'acide borique.

6. Procédé de préparation d'alcools orthohydroxybenzyliques selon l'une quelconque des revendications 1 à 5, caractérisé en ce que la température de la réaction de condensation est comprise entre 20 et 120 °C.

7. Procédé de préparation d'alcools orthohydroxybenzyliques selon l'une quelconque des revendications 1 à 6, caractérisé en ce que les phénols en excès sont séparés des alcools orthohydroxybenzyliques par traitement de la masse réactionnelle de condensation par une solution aqueuse d'une base alcaline à raison de 0,8 à 1,2 mole de base alcaline par mole d'acide borique mis en œuvre, séparation après décantation d'une solution organique contenant les phénols de la solution aqueuse contenant un sel alcalin complexe de l'alcool orthohydroxybenzylique et de l'acide borique formé puis libération de ce dernier de son complexe par tout moyen approprié.

8. Procédé de préparation d'alcools orthohydroxybenzyliques selon la revendication 7, caractérisé en ce que la quantité de base alcaline est comprise entre 0,9 et 1,1 mole par mole d'acide borique.

9. Procédé de préparation d'alcools orthohydroxybenzyliques selon la revendication 7, caractérisé en ce que la quantité de base alcaline est de 1 mole par mole d'acide borique.

10. Procédé de préparation d'alcools orthohydroxybenzyliques selon la revendication 7, caractérisé en ce que la base alcaline est la soude ou la potasse.

11. Procédé de préparation d'alcools orthohydroxybenzyliques selon la revendication 7, caractérisé en ce que l'alcool orthohydroxybenzylique est libéré par traitement de la phase aqueuse du complexe alcalin par une base alcaline avec formation d'un borate alcalin et du sel alcalin de l'alcool orthohydroxybenzylique.

12. Procédé de préparation d'alcools orthohydroxybenzyliques selon l'une quelconque des revendications 1 à 11, caractérisé en ce que l'on prépare des alcools orthohydroxybenzyliques de formule :

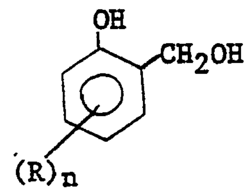

dans laquelle n est 0 ou un nombre entier de 1 à 3, R représente un radical alcoyle ayant de 1 à 12 atomes de carbone ; un radical alcoyloxyle ayant de 1 à 12 atomes de carbone ; un atome d'halogène, à partir de phénols de formule générale :

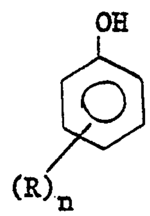

dans laquelle n et R ont la signification donnée ci-avant, l'une au moins des positions en ortho du groupe phénolique étant libre quand n est 2 ou 3.

13. Procédé de préparation de saligénol selon l'une quelconque des revendications 1 à 11, caractérisé en ce que l'on fait réagir le formaldéhyde ou un générateur de formaldéhyde sur une solution dans un solvant organique d'un borate de phényle obrenu par réaction de 1,1 à 3 moles de phénol avec une mole d'acide borique dans un solvant organique formant un azéotrope avec l'eau de réaction et élimination de cette dernière par distillation azéotropique, à raison d'environ 1 mole de formaldéhyde par mole d'acide borique, entre 20 et 120 °C puis que l'on traite la masse réactionnelle par 0,8 à 1,2 mole d'une base alcaline en solution aqueuse par mole d'acide borique, sépare la couche organique de phénol de la phase aqueuse contenant le sel alcalin du saligénol et de l'acide borique et enfin que l'on traite cette phase aqueuse par une solution aqueuse de base alcaline pour libérer le saligénol sous forme de saligénate alcalin.

**Claims**

1. Process for the preparation of ortho-hydroxybenzyl alcohols by reacting esters of boric acid and of phenols with formaldehyde or a formaldehyde-generating substance, with the formation of boric acid esters of ortho-hydroxybenzyl alcohols, and then decomposing the latter in order to liberate the ortho-hydroxybenzyl alcohols, characterised in that the boric acid esters of phenols used for the condensation are obtained by reacting at least 1.1 mols of a phenol with one mol of boric acid.

2. Process for the preparation of ortho-hydroxybenzyl alcohols, according to Claim 1, characterised in that the phenol borates have been obtained by reacting 1.1 to 3 mols of a phenol with 1 mol of boric acid.

3. Process for the preparation of ortho-hydroxybenzyl alcohols, according to any one of Claims 1 to 2, characterised in that the phenol borates are in solution in the solvent used during the esterification of the phenol with boric acid.

4. Process for the preparation of ortho-hydroxybenzyl alcohols, according to Claim 3, characterised in that the solvent is an aromatic hydrocarbon.

5. Process for the preparation of ortho-hydroxybenzyl alcohols, according to any one of Claims 1 to 4, characterised in that the amount of formaldehyde employed is of the order of 1 mol per mol of boric acid.

6. Process for the preparation of ortho-hydroxybenzyl alcohols, according to any one of Claims 1 to 5, characterised in that the temperature of the condensation reaction is between 20 and 120 °C.

7. Process for the preparation of ortho-hydroxybenzyl alcohols, according to any one of Claims 1 to 6, characterised in that the excess phenols are separated from the ortho-hydroxybenzyl alcohols by treating the condensation reaction mixture with an aqueous solution of an alkali metal base at the rate of 0.8 to 1.2 mols of alkali metal base per mol of boric acid employed, separating, by decantation, an organic solution containing the phenols from the aqueous solution containing the resulting complex alkali metal salt of the ortho-hydroxybenzyl alcohol and of boric acid, and then liberating the ortho-hydroxybenzyl alcohol from its complex by any suitable means.

8. Process for the preparation of ortho-hydroxybenzyl alcohols, according to Claim 7, characterised in that the amount of alkali metal base is between 0.9 and 1.1 mol per mol of boric acid.

9. Process for the preparation of ortho-hydroxybenzyl alcohols, according to Claim 7, characterised in that the amount of alkali metal base is 1 mol per mol of boric acid.

10. Process for the preparation of ortho-hydroxybenzyl alcohols, according to Claim 7, characterised in that the alkali metal base is sodium hydroxide or potassium hydroxide.

11. Process for the preparation of ortho-hydroxybenzyl alcohols, according to Claim 7, characterised in that the ortho-hydroxybenzyl alcohol is liberated by treating the aqueous phase of the alkali metal complex with an alkali metal base, with the formation of an alkali metal borate and the alkali metal salt of the ortho-hydroxybenzyl alcohol.

12. Process for the preparation of ortho-hydroxybenzyl alcohols, according to any one of Claims 1 to 11, characterised in that ortho-hydroxybenzyl alcohols of the formula :

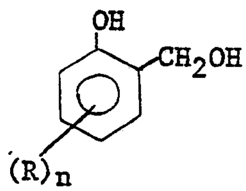

in which n is 0 or an integer from 1 to 3 and R represents an alkyl radical having from 1 to 12 carbon atoms, an alkoxy radical having from 1 to 12 carbon atoms or a halogen atom, are prepared from phenols of the general formula :

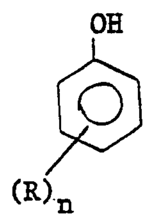

in which n and R have the meaning given above, at least one of the ortho-positions relative to the phenol group being free when n is 2 or 3.

13. Process for the preparation of saligenol, according to any one of Claims 1 to 11, characterised in that formaldehyde or a formaldehyde generator is reacted with a solution, in an organic solvent, of a phenyl borate obtained by reacting 1.1 to 3 mols of phenol with one mol of boric acid in an organic solvent which forms an azeotrope with the water of reaction, and removing the latter by azeotropic distillation, at the rate of about 1 mol of formaldehyde per mol of boric acid and at between 20 and 120 °C, in that the reaction mixture is then treated with 0.8 to 1.2 mols of an alkali metal base in aqueous solution per mol of boric acid, the organic layer of phenol is separated from the aqueous phase containing the alkali metal salt of saligenol and of boric acid, and in that this aqueous phase is finally treated with an aqueous solution of an alkali metal base in order to liberate the saligenol in the form of an alkali metal saligenate.

**Ansprüche**

1. Verfahren zur Herstellung von Orthohydroxybenzylakoholen durch Reaktion von Borsäure-Estern von Phenolen mit Formaldehyd oder einem Formaldehyd bildenden Körper unter Bildung von Borestern von Orthohydroxybenzylalkoholen und dann Zersetzung dieser letzteren zur Freisetzung der Orthohydroxybenzylalkohole, dadurch gekennzeichnet, daß die zur Kondensation verwendeten Borester von Phenolen erhalten sind durch Reaktion von mindestens 1,1 Mol eines Phenols mit 1 Mol Borsäure.

2. Verfahren zur Herstellung von Orthohydroxybenzylalkoholen gemäß Anspruch 1, dadurch gekennzeichnet, daß die Phenol-Borate erhalten wurden durch Reaktion von 1,1 bis 3 Mol eines Phenols mit 1 Mol Borsäure.

3. Verfahren zur Herstellung von Orthohydroxybenzylalkoholen gemäß einem der Ansprüche 1 bis 2, dadurch gekennzeichnet, daß die Borate von Phenolen in Lösung in dem Lösungsmittel sind, welches bei der Veresterung des Phenols durch die Borsäure verwendet wurde.

4. Verfahren zur Herstellung von Orthohydroxybenzylalkoholen gemäß Anspruch 3, dadurch gekennzeichnet, daß das Lösungsmittel ein aromatischer Kohlenwasserstoff ist.

5. Verfahren zur Herstellung von Orthohydroxybenzylalkoholen gemäß einem der Ansprüche 1 bis 4, dadurch gekennzeichnet, daß die eingesetzte Formaldehydmenge nahe bei 1 Mol pro Mol Borsäure liegt.

6. Verfahren zur Herstellung von Orthohydroxybenzylalkoholen gemäß einem der Ansprüche 1 bis 5, dadurch gekennzeichnet, daß die Temperatur der Kondensationsreaktion zwischen 20 und 120 °C liegt.

7. Verfahren zur Herstellung von Orthohydroxybenzylalkoholen gemäß einem der Ansprüche 1 bis 6, dadurch gekennzeichnet, daß die überschüssigen Phenole von den Orthohydroxybenzylalkoholen abgetrennt werden durch Behandlung der Kondensationsreaktionsmasse mit einer wässrigen Lösung einer Alkali-Base in einer Menge von 0,8 bis 1,2 Mol Alkali-Base pro mol eingesetzte Borsäure, Abtrennung nach Dekantieren einer organischen Lösung, welche die Phenole enthält, von der wässrigen Lösung, welche ein komplexes Alkali-Salz des Orthohydroxybenzylalkohols und der gebildeten Borsäure enthält, dann Freisetzung dieses letzteren aus seinem Komplex durch jedes geeignete Mittel.

8. Verfahren zur Herstellung von Orthohydroxybenzylalkoholen gemäß Anspruch 7, dadurch gekennzeichnet, daß die Menge an alkalischer Base zwischen 0,9 und 1,1 Mol pro Mol Borsäure beträgt.

9. Verfahren zur Herstellung von Orthohydroxybenzylalkoholen gemäß Anspruch 7, dadurch gekennzeichnet, daß die Menge an Alkali-Base 1 Mol pro Mol Borsäure beträgt.

10. Verfahren zur Herstellung von Orthohydroxybenzylalkoholen gemäß Anspruch 7, dadurch gekennzeichnet, daß die Alkali-Base Natriumhydroxid oder Kaliumhydroxid ist.

11. Verfahren zur Herstellung von Orthohydroxybenzylalkoholen gemäß Anspruch 7, dadurch gekennzeichnet, daß der Orthohydroxybenzylalkohol freigesetzt wird durch Behandlung der wässrigen Phase des Alkali-Komplexes mit einer Alkali-Base unter Bildung eines Alkali-Borats und des Alkali-Salzes des Orthohydroxybenzylalkohols.

12. Verfahren zur Herstellung von Orthohydroxybenzylalkoholen gemäß einem der Ansprüche 1 bis 11, dadurch gekennzeichnet, daß man Orthohydroxybenzylalkohole der Formel

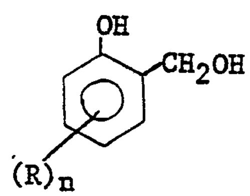

worin n gleich 0 oder eine ganze Zahl von 1 bis 3 ist, R einen Alkylrest mit 1 bis 12 Kohlenstoffatomen ; einen Alkyloxyrest mit 1 bis 12 Kohlenstoffatomen ; ein Halogenatom bedeutet, herstellt ausgehend von Phenolen der allgemeinen Formel

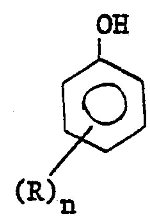

worin n und R die oben angegebene Bedeutung besitzen, wobei wenigstens eine der Stellungen in ortho-Stellung der Phenylgruppe frei ist, wenn n = 2 oder 3.

13. Verfahren zur Herstellung von Saligenol gemäß einem der Anspüche 1 bis 11, dadurch gekennzeichnet, daß man Formaldehyd oder einen Formaldehyderzeuger zur Reaktion bringt mit einer Lösung eines Phenylborats in einem organischen Lösungsmittel, erhalten durch Reaktion von 1,1 bis 3 Mol Phenol mit 1 Mol Borsäure in einem organischen Lösungsmittel, das mit dem Reaktionswasser ein Azeotrop bildet und Entfernung dieses letzteren durch azeotrope Destillation, in einer Menge von etwa 1 Mol Formaldehyd pro Mol Borsäure, zwischen 20 und 120 °C, und daß man dann die Rekationsmasse mit 0,8 bis 1,2 Mol einer Alkali-Base in wässriger Lösung pro Mol Borsäure behandelt, die organische Phenolschicht von der wässrigen Schicht, welche das Alkali-Salz des Saligenols und der Borsäure enthält, abtrennt, und daß man schließlich diese wässrige Phase mit einer wässrigen Lösung alkalischer Base behandelt, um das Saligenol in Form des Alkali-Saligenats freizusetzen.